# EUROPEAN PATENT SPECIFICATION

(11) **EP 0 300 033 B1**
(45) Date of publication and mention of the grant of the patent: **17.11.1994**
(21) Application number: 88902625.8
(22) Date of filing: 13.01.1988
(51) Int. Cl.: C07K 15/00, A61K 37/48

(54) **CYTOTOXIC LYMPHOCYTE-SPECIFIC PROTEASE-RELATED MOLECULES AND METHODS**
PROTEASE-ÄHNLICHE ZYTOTOXISCHE LYMPHOZYTEN-SPEZIFISCHE MOLEKÜLE UND VERFAHREN
MOLECULES RELATIVES AUX PROTEASES ET SPECIFIQUES DES LYMPHOCYTES CYTOTOXIQUES

(30) Priority: 13.01.1987 US 2960
(43) Date of publication of application: 25.01.1989
(73) Proprietor: SERAGEN, INC., Hopkinton, MA 01748 (US)
(72) Inventor: BLEACKLEY, Robert, C., Edmonton, Alberta T6G 1R9 (CA); LOBE, Corrine, G., Edmonton, Alberta P5A 0V2 (CA); PAETKAU, Verner, H., Edmonton, Alberta P6E 1S6 (CA); JAMES, Michael, N., G., Edmonton, Alberta P6G 1X1 (CA); MURPHY, Michael, Edmonton, Alberta T6J 0A2 (CA)
(74) Representative: Robinson, Anthony John Metcalf
(86) International application number: US8800071
(87) International publication number: WO8805471

(56) References cited:
- PROC. NATL. ACAD. SCI. USA, vol. 83, issued 1986 March (Washington, D. C.), C.G. Lobe, et al., pp. 1448-1452
- SCIENCE, vol. 232, issued 16 May 1986 (Washington, D.C.), C.G. Lobe et al., pp. 858-861
- NATURE, vol. 314, issued 25 April 1985 (London), M.S. Pasternack et al., pp. 743-745
- J. BIOL. CHEM., vol. 261, no. 3, issued 25 Juanary 1986 (Washington, D.C.), H.C. Cheng et al., pp. 989-992
- CANCER RES., vol. 46, issued August 1986 (Baltimore, MD), L.E. Ostrowski et al., pp. 4121-4128
- FEBS LETT., vol. 197, no. 1, 2, issued 3 March 1986 (Amsterdam), C. Betzel et al., pp. 105-110
- NATURE, vol. 322, issued 21 August 1986 (London), M.S. Pasternack et al., pp. 740-743
- SCIENCE, vol. 232, issued 16 May 1986 (Washington, D.C.), H.K. Gershenfeld et al., pp. 740-743
- BIOCHEM. BIOPHYS. ACTA, vol. 452, 8 Dec.1976 (Amsterdam), K. Poulsen et al., pp. 533-537

## Description

### Background of the Invention

This invention relates to protease inhibitors.

Thymus derived (T) lymphocytes play a major role in the immune system. Maturation of the T cell lineage involves three distinct stages: (a) generation of a T cell precursor from a pluripotent stem cell, (b) differentiation in the thymus, and (c) migration of mature cells to the peripheral tissues. Maturation of T cells within the thymus is antigen independent. However, once they have left the thymus, upon interaction with an antigen they are driven through the final steps of differentiation to become mature cells. These final steps are complex and involve interactions with other cells and soluble effector molecules.

Several subsets of T cells have been recognized among activated peripheral T cells. There are three main classes: helper, suppressor, and cytotoxic. Helper T lymphocytes potentiate immune responses (both humoral and cell-mediated) either by cell-cell contact or by synthesis and secretion of factors. These factors, although synthesized in response to an antigen-specific signal, can be either antigen-specific or antigen-nonspecific. Suppressor T lymphocytes inhibit the functions of other lymphocytes, again either directly or via soluble factors. Cytotoxic T lymphocytes are the effector cells in cell mediated immune reactions. They specifically recognize foreign antigens on the surface of cells, bind to them, and cause the target cell to lyse. Cytotoxic T lymphocytes are known to cause or exacerbate autoimmune diseases such as rheumatoid arthritis, and are also involved in allograft rejection and graft-versus-host disease.

The various steps in the process of cytotoxic T lymphocyte induced lysis have been analyzed in some detail, e.g., Berke, 72 Immunol. Rev. 5 (1983); Nabholz & MacDonald, l Ann. Rev. Immunol. 273 (1983). Recent studies by Podack & Konigsberg, 160 J. Exp. Med. 695 (1984) and Henkart et al., 160 J. Exp. Med. 75 (1984) have suggested that the dense cytoplasmic granules seen in CTL and natural killer cells are directly involved in target cell lysis by a mechanism involving transmembrane channels.

A general description of cytotoxic T lymphocytes, natural killer cells, and killer (K) cells is contained in Stites et al., Basic & Clinical Immunology 227-31 (Lange Medical Publications, Los Altos, Ca., 1984).

Science, vol. 232 (1986), 858-861 discloses two cDNAs, B10 and C11, encoding serine proteases and specifically expressed by cytotoxic T lymphocytes.

### Summary of the Invention

The invention features, in a first aspect, a molecule capable of inhibiting the enzymatic activity of a protease produced by a cytotoxic lymphocyte in a mammal to which the molecule is administered, the peptide being incapable of inhibiting plasmin, and the enzymatic activity of the protease being to cleave a protein between the C-linkage of an aspartic acid residue of the protein and the N-linkage of an adjacent amino acid residue of the protein.

Cytotoxic lymphocytes include cytotoxic T lymphocytes, natural killer cells, and killer (K) cells. (Plasmin, an enzyme important in the blood coagulation pathway, is specified herein as an example of a physiologically important enzyme not adversely affected by the inhibitor molecules of the invention which, as is explained in more detail below, are very specific.)

The term mammal herein includes a human.

In preferred embodiments, the peptide has 20 or fewer amino acid residues, and the protease is a serine protease. In other preferred embodiments, the protease is produced exclusively in the mammal by cytotoxic lymphocytes, and the molecule exclusively inhibits proteases produced by cells of the mammal which are cytotoxic lymphocytes.

The term competitive inhibition, as used herein, refers to inhibition in which the inhibitor combines with the free protease such that it competes with the normal substrate of the protease. Competitive inhibition is described, e.g., in Lehninger, Biochemistry 197-200 (Worth, 2d ed. 1975).

The term protease, as used herein, refers to an enzyme that hydrolises, and thus cleaves, peptide bonds.

The term serine protease, as used herein, refers to a protease which has a serine residue at the active site of the enzyme.

A second aspect relates to the use of a peptide molecule of the first aspect in the preparation of an agent for the treatment of allograft rejection or an autoimmune disease or disorder.

The allograft rejection, autoimmune disease or disorder is characterised by the production of the protease by cytotoxic lymphocytes, which is the cause of such ailments.

The invention contemplates the use of a first gene encoding a polypeptide ligand capable of binding exclusively to an unwanted cell and a second gene encoding a protease produced by the cytotoxic lymphocytes of a mammal, the protease being involved in the cytolytic process of the mammal; where the first gene and second gene can be fused and the fused gene can be cloned into a vector; a microorganism can be transformed with the vector and the transformed microorganism can be grown so that a hybrid protein can be isolated from the microorganism, where the hybrid protein comprises the polypeptide ligand and the protease; in the preparation of an agent in the treatment of a disorder characterised by the presence of an unwanted cell.

The invention additionally envisages a method of monitoring a mammal having a disorder characterised by the expression of, in cytotoxic lymphocytes of the mammal, a gene encoding a protease, the gene being exclusively expressed by the cytotoxic lymphocytes in the mammal; the method comprising the steps of:
providing a probe sequence capable of hybridising to the gene;
providing a nucleic acid sample of the mammal;
contacting the probe sequence with the sample to allow hybridisation of the probe sequence to the gene; and
detecting the hybridisation.

A third aspect of the present invention relates to the use of the peptide molecule of the first aspect for use in medicine.

A fourth aspect relates to a method of measuring the cytotoxicity of a cytotoxic lymphocyte of a mammal, the method comprising:
providing the nucleic acid probe B10 or C11 which is capable of hybridising to a gene of a killer cell, the gene being exclusively expressed by cytotoxic lymphocytes of the mammal and encoding a protease which cleaves a protein, the cleavage site being between the C-linkage of an aspartic residue of the protein and the N-linkage of an adjacent residue of the protein;
contacting the probe sequence with the sample to allow hybridisation of the probe sequence to the gene; and
detecting hybridisation.

A fifth aspect concerns a pharmaceutical composition comprising a peptide molecule of the first aspect and a pharmaceutically acceptable carrier.

The molecule may be prepared by coupling successive amino acid residues together. The pharmaceutical composition may be prepared by admixing the molecule of the first aspect with a pharmaceutically acceptable carrier.

Preferred features of one aspect of the present invention are as for another aspect *mutatis mutandis*.

The term peptide, as used herein, includes proteins as well as peptides too short to be characterized as proteins. Generally those peptides having a molecular weight of greater than than 5,000 are characterized as proteins.

Cytotoxic lymphocytes produce, as part of their cytotoxic activity, proteases, some of which, we have discovered, cleave proteins at sites different from the sites cleaved by proteases such as plasmin produced by other cells of the body. The molecules of the invention, since they mimic these unite cleavage sites, can exclusively inhibit those proteases produced by cytotoxic lymphocytes. Thus a person suffering from an immune disorder, or experiencing allograft rejection, can be administered a molecule of the invention to inhibit the cytotoxic lymphocytes involved in the disease or rejection process, and the administered molecule will not interfere with, for example, lysis of blood clots, or other normal protease-dependent functions.

### Description of the Preferred Embodiment

The structure, synthesis, and use of the preferred embodiments are discussed next, after the drawings are briefly described.

### Drawings

Fig. 1 is a group of photographs of the cytodots of various cell lines hybridized with a labeled protease-encoding cDNA.

Fig. 2 is a group of photographs of blot-hybridizations
of three protease-encoding clones with various cell lines.

Fig. 3 is a graph showing the correlation of a protease mRNA expression (●) with cell activation in a mixed lymphocyte culture.

Fig. 4 is a partial nucleotide sequence comparison of two protease-encoding cDNA's.

Fig. 5 is the nucleotide sequence of one of said cDNA's and the predicted protein structure it encodes.

Fig. 6 is a partial amino acid sequence comparison of five serine proteases.

### Structure

Cytotoxic lymphocytes synthesize a characteristic set of cytotoxicity-related proteases which are expressed at much reduced levels, if at all, in other subsets of lymphocytes.

The cytotoxicity-related proteases can be divided into two groups, effector proteases and non-effector proteases. Effector proteases are released by a cytotoxic lymphocyte when it comes in contact with a target cell, and break down proteins in the membrane of the target cell or enter the target cell and hydrolyze intracellular proteins, leading to the cell's destruction. Non-effector proteases are involved in the enzymatic processes that lead to the production and/or release of the effector proteases (or other effector molecules) from the lymphocyte. Inhibiting the action of either an effector protease or a non-effector protease inhibits the ability of cytotoxic lymphocytes to destroy a target cell.

The molecules of the invention competitively inhibit the activity of proteases produced by cytotoxic lymphocytes, while not inhibiting the activity of proteases produced by other cell types, including any proteases produced by both cytotoxic lymphocytes and other cells. Preferably the inhibitory molecules are peptides.

The structure of the preferred inhibiting peptides can be determined by analyzing the protease to ascertain its active site and the corresponding cleavage site of target proteins. This can be done using standard computer techniques well known to those skilled in the art using conventional software and hardware, for example, by using the program MMS (available from the Dept. of Chem., UCSD, San Diego, Ca.) with a Silicon Graphics work station (available from Silicon Graphics Computer Systems, Mountain View, Ca.) or by using FRODO (available from the Dept. of Chem., UCSD, San Diego, Ca.) or MIDAS (available from the Dept. of Pharm. Chem., UCSF, San Fransisco, Ca.) on the Evans and Sutherland PS300 system (available from Evans and Sutherland Co., Salt Lake City, Utah).

The preferred peptides contain the two amino acids that constitute the cleavage site recognized by the protease, and have between 3 and 20 (more preferably between 3 and 5) amino acid residues. Shorter peptides are preferred because they are, in general, readily taken up by cells. The peptides should not contain a cleavage site recognized by proteases produced by cells other than cytotoxic lymphocytes, for example, those sites described by Zreighton, Proteins: Structure and Molecular Properties 336-37, 427-38 (W.H. Friedman, N.Y., 1983).

Prior to the above analysis, the amino acid sequence of the protease, at least in the region of the putative active site, must be determined. The general procedure is, first, to obtain a DNA sequence (a gene or cDNA) that is expressed only in cytotoxic lymphocytes, e.g., by conventional differential hybridization analysis; second, to sequence the gene; and, third, to deduce the amino acid sequence of the protein. DNA sequences encoding proteins which from their structure appear to be proteases (preferably serine proteases) are selected for further analysis. Those which recognize cleavage sites of proteins different from the cleavage sites recognized by other non-cytotoxic lymphocyte-produced proteases are the preferred proteases of this invention.

Described in Example 1 below is the isolation, cloning, and characterization of two genes expressed exclusively in the cytotoxic T lymphocytes of mice. (Exclusively means that either the genes are not expressed, or are only expressed in very low (less than 5 molecules of mRNA per cell) levels, in other types of cells in the organism). Example 2 describes the sequencing of the two genes, the determination of the amino acid sequence of the protease which one of the genes encodes, and the characteristics of the protease. Example 3 describes the three dimensional structure of the protease, and the structure of a peptide that can act as a competitive inhibitor of the protease. Example 4 describes the procedure that can be followed to obtain proteases produced exclusively by human cytoxic T lymphocytes.

### Example 1

Cells - The cytotoxic T-cell lines MTL2.8.2 and MTL11.1 were generated from CBA/J mice as described by Bleackley et al., 128 J. Immunol. 758 (1982). EL4.E1 is an interleukin 2 (IL-2)-producing variant of the EL4 cell line described by Farr et al., 125 J. Immunol. 2555 (1980). CHl is a CBA/J α CBA/J X BALB/c antigen-specific helper T-cell line. It was produced from a 2-day mixed lymphocyte culture by continuous restimulation with irradiated F₁ spleen cells in RPMI 1640 medium supplemented with 10% fetal bovine serum and 100 µM 2-mercaptoethanol (RHFM). To generate human cytotoxic T lymphocytes (CTL), peripheral blood lymphocytes were incubated in RHFM and stimulated with irradiated allogeneic cells at days 0 and 7 and harvested at day 10. The fetal-derived cells used are described by Teh et al., 135 J. Immunol. 1582 (1985). For the time course of cell activation, spleen cells from CBA/J mice were incubated in RHFM (10⁶ cells per ml) and purified IL-2 (described by Riendeau et al., 258 J. Biol. Chem. 12l14 (1983)), either with an equal number of mitomycin C-treated EL4.E1 cells or Con A (2 µg/ml). Samples were removed at day 1 through day 6, assayed for cytotoxic activity by the procedure described in Shaw et al., 120 J. Immunol. 1974 (1978), and analyzed by cytodot hybridization.

cDNA Library Construction - Double-stranded cDNA was synthesized from 4 µg of MTL2.8.2 mRNA as described by Gubler and Hoffman, 25 Gene 263 (1983). Following repair with the Klenow fragment of DNA polymerase and T4 DNA polymerase to maximize flush ends, phosphorylated EcoRI linkers (P-L Biochemicals) were ligated to the cDNA in 70 mM Tris-HCl, pH 7.6/10 mM MgCl₂/5 mM dithiothreitol/1 mM ATP/1 unit of T4 DNA ligase at 14°C overnight (Goodman & MacDonald, 68 Methods Enzymol. 75 (1979)). After digestion with EcoRI, the product was run on a 5-ml Sepharose® 4B column, and the excluded fractions were pooled and ethanol-precipitated. The cDNA was ligated to EcoRI/bacterial alkaline phosphatase-treated pUC13 (P-L Biochemicals) in 66 mM Tris-HCl, pH 7.6/6.6 mM MgCl₂/10 mM dithiothreitol/1 mM ATP. Reactions were heated to 37°C for 5 min, quick-chilled before the addition of 1 unit of T4 DNA ligase, and incubated at 14°C for 2 hr. Escherichia coli JM83 cells were made competent by using the CaCl₂/RbCl procedure described by Maniatis et al. in Molecular Cloning: A Laboratory Manual (Cold Spring Harbor Laboratory, Cold Spring Harbor, N.Y., 1982) and were transformed with the ligated cDNA. White colonies (those containing inserts) were ordered in 96-well microtiter plates and stored in LB medium containing 20% glycerol at -70°C.

Differential Screening - Colonies were replicated in triplicate onto nitrocellulose filters, grown for 6 hr, and then amplified on chloramphenicol (100 µg/ml) for 12 hr. Bacteria were lysed, and the filters were prewashed to remove bacterial debris, as described by Maniatis, supra. Prehybridization at 42°C for 12-20 hr was done in 50% (vol/vol) formamide containing 2x Denhardt's solution (1x Denhardt's solution = 0.02% polyvinylpyrrolidone/0.02% Ficoll/0.02% bovine serum albumin), 4x SET buffer (1x SET buffer = 0.6 M NaCl/0.12 M Tris-HCl, pH 8/1 mM EDTA), 0.1% NaDodSO₄, 100 µg of yeast tRNA per ml, and 125 µg of poly(A) per ml (Sigma). Hybridization in the same buffer included 1-5 x 10⁵ cpm of cDNA probe per ml synthesized from mRNA with 20 µg of T-primers per ml (Collaborative Research, Waltham, MA); 50 mM Tris-HCl (pH 8.3); 10 mM MgCl₂; 5 mM dithiothreitol, 500 µM each of dGTP, dATP, and dTTP; 70 mM KCl; 30 µCi (1 Ci = 37 GBq) of [α-³²P]dCTP (New England Nuclear, 300 Ci/mmol); and 15 units of avian myeloblastosis virus reverse transcriptase at 42°C for 60 min. Template RNA was hydrolyzed by the addition of NaOH to 1.5 M. Samples were boiled for 3 min and fractionated by Sephadex® G-50 column chromatography. Filters were washed in 5x SET buffer for 15 min at 22°C and then in 2x SET buffer/50% formamide for 20 min at 42°C and were exposed to film (Kodak X-Omat® AR) with an intensifying screen for 1 to 3 days at -70°C. Hybridized probe was removed by boiling the filters for 10 min in distilled water.

Blot Analysis - Cytodots were prepared as described by White and Bancroft, 257 J. Biol. Chem. 8569 (1982). For blot-hybridization analysis, total cytoplasmic RNA (10 µg) or poly(A)⁺ mRNA (2 µg) was denatured in 6.3% formaldehyde/50% formamide at 55°C and size fractionated on a 0.8% agarose gel containing 0.66% formaldehyde. RNA was transferred to nitrocellulose as described by Thomas, 77 Proc. Natl. Acad. Sci. USA 5201 (1980). Plasmid DNA was digested with EcoRI, run on a 0.7% agarose gel, and transferred to nitrocellulose, as described by Southern, 26 J. Mol. Biol. 365 (1975). Filters were baked at 80°C for 2 hr, then prehybridized at 42°C for 6-12 hr in 50% formamide containing 20 mM phosphate buffer (pH 6.8), 2 mM pyrophosphate, 100 µM ATP, 5x Denhardt's solution, 0.75 M NaCl, 0.075 M sodium citrate (pH 7), 100 µg of salmon sperm DNA per ml, 0.1% NaDodSO₄, 50 µg of poly(A) per ml, and 2.5 mM EDTA. Hybridization was carried out in the same buffer with a nick-translated plasmid of specific activity 1 x 10⁸ cpm/µg (Bethesda Research Laboratories kit) at 1 x 10⁶ cpm/ml.

Results - Triplicate copies of the library were hybridized first with cDNA synthesized from MTL2.8.2 mRNA, then, after autoradiography and washing, with helper T-cell cDNA, and finally with thymocyte cDNA. Colonies that gave a higher hybridization signal with killer cell mRNA in at least two of the three copies of the library were picked. Upon rescreening, again in triplicate, 36 of these 121 colonies appeared to be clearly CTL-specific. Plasmid DNA isolated from these colonies was cut with EcoRI, and a series of cross-hybridizations was performed. Two clones were chosen for more extensive analysis: clone B10 because it appeared to be the most abundant in the library, cross-hybridizing strongly with eight other inserts, and clone C11 because it weakly cross-hybridized with B10 but not with all B10-related clones (one other C11-related sequence was found).

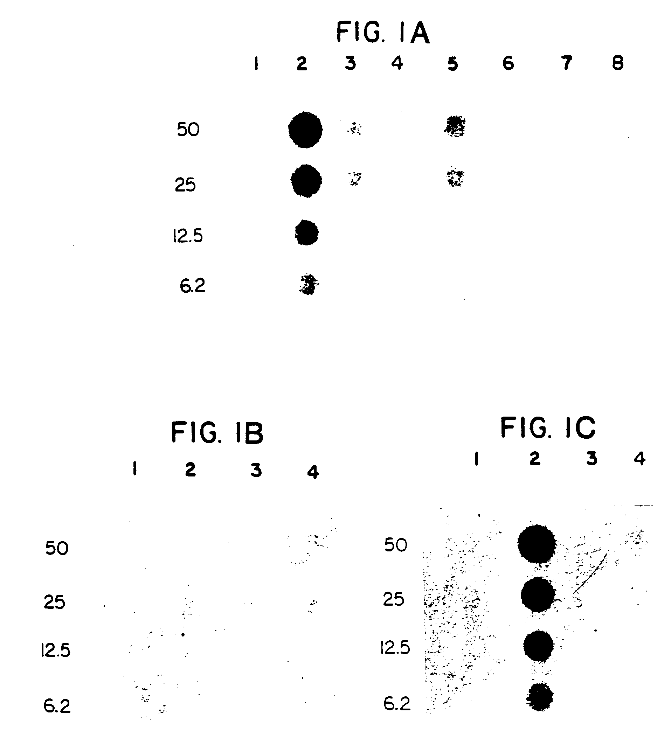

Cytodots prepared from a variety of cells and tissues were hybridized with nick-translated B10 and C11. Results are shown for B10 (Fig. 1A). The number of cells x 10⁴ is shown. The data with probe C11 are similar and are not shown. The highest signal was detected in MTL2.8.2 (lane 2)--i.e., the killer cell line that was used to generate the cDNA library. A weaker but positive signal was observed with MTL-III (lane 3), a variant of MTL2.8.2 that had a low level of cytotoxicity and had become IL-2 and antigen independent. A similar level of expression was observed in a novel T-cell clone (lane 5) derived from murine fetal thymus of Teh, supra. There was no evidence for expression in either mouse thymocytes (lane 1) or a helper T-cell line (CH1) (lane 6) that secretes IL-2 in response to antigen. Mouse brain (lane 7) and liver (lane 8) and human CTL line (lane 4) were similarly negative under the high-stringency conditions of this experiment. In addition to the data given in Fig. 1, no evidence for expression of B10 or C11 was found in either a sample of enriched natural [human?] killer cells (Kumagai et al., 129 J. Immunol. 388 (1982)), even after interferon stimulation (Dejeu et al., 122 J. Immunol. 175 (1979)), or a helper T-cell hybridoma that secretes an antigen-specific factor (Kwong et al. 133 J. Immunol. 653 (1984)). To ensure that the negative samples did contain hybridizable RNA, all of the cytodots were reprobed with either a lymphocyte-specific probe or oligo(dT) or the T-cell antigen receptor β-chain gene (Hendrick et al., 308 Nature (London) 153 (1984)). Although the level of signal varied, all samples were positive.

To enrich for the B cells of a spleen cell suspension, lymphocytes were separated from adherent cells on Petri dishes and then treated with anti-Thy-1.2 antiserum. The enriched B cells were then incubated with lipopolysaccharide (LPS) or Con A or RHFM medium. After 24 hr, the cells were harvested, cytodots were prepared (lane 1, no incubation or additive; lane 2, incubation with LPS; lane 3, incubation with Con A; lane 4, incubation with RHFM medium), and the filter was probed with B10 or C11 (Fig. 1B; only the results with B10 shown, as the results with C11 were the same). No expression of either sequence could be detected in any sample. However, when the blot was hybridized with an immunoglobulin µ heavy chain probe (Calame et al., 284 Nature (London) 452 (1980)), a stronge positive signal was seen in the LPS-stimulated cells (lane 2) (Fig. 1C).

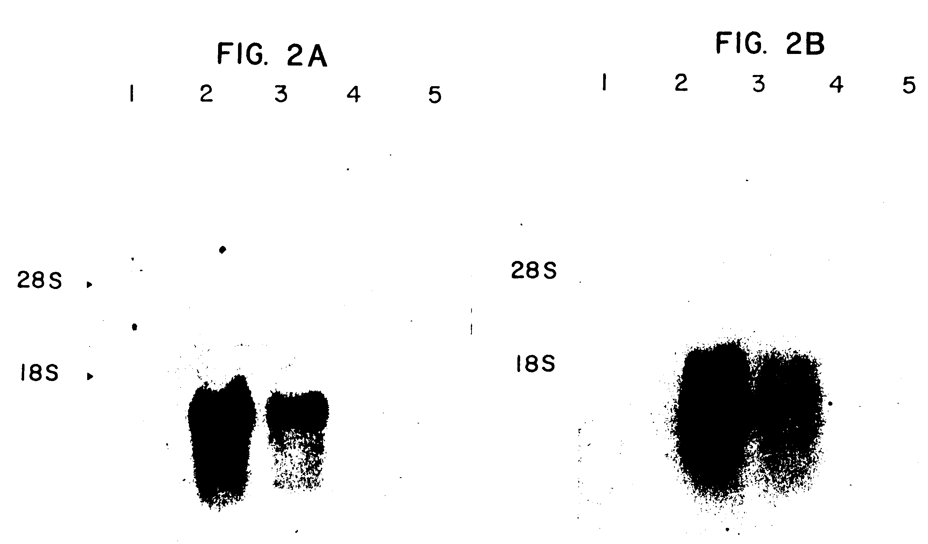

Poly(A)⁺ RNA was isolated from a variety of cell sources, run on a denaturing agarose gel, and transfered to nitrocellulose. Fig. 2 shows the same filter probed first with nick-translated 810 (Fig. 2A), then with C11 (Fig. 2B), and finally with probe 10 (results not shown), a cloned gene that detects mRNA in a variety of cell types (Paetkau et al., in 10 Contemporary Topics in Molecular Biology 35 (S. Gillis ed., Plenum, N.Y., 1984)). Probe B10 detected a single band (approximately 900 bases) in two different murine cytotoxic T cell clones, MTL2.8.2 (Fig. 2, lane 2) and MTL11.1 (Fig. 2, lane 3). No bands were detected in RNA from thymocytes (Fig. 2, lane 1), an antigen-specific helper cell line (Fig. 2, lane 4), or murine thymoma EL4 (Fig. 2, lane 5). When the blot was reprobed with C11, again only the two cytotoxic T cell clones showed bands. However, in contrast to B10, this probe hybridized to two bands, one of approximately 900 bases and the other of 1200 bases. Probe 10 detected a band in all cell samples. In addition to the results shown in Fig. 1, blot-hybridization analysis was performed on poly(A)⁺ RNA from a number of murine cells including eleven CTL lines, two helper lymphocyte lines, brain, liver, three helper T-cell lines, unstimulated and LPS-stimulated B lymphocytes, and one B-cell myeloma. Of these, only the actively cytotoxic T cells expressed mRNAs that hybridized with B10 and C11. To ensure that all tracks contained hybridizable RNA, the blot was rehybridized with probe 10. A band of the expected size was seen in all tracks.

The results from the cytodots and blot-hybridization analysis indicates that both B10 and C11 are murine cytotoxic Tlymphocyte specific.

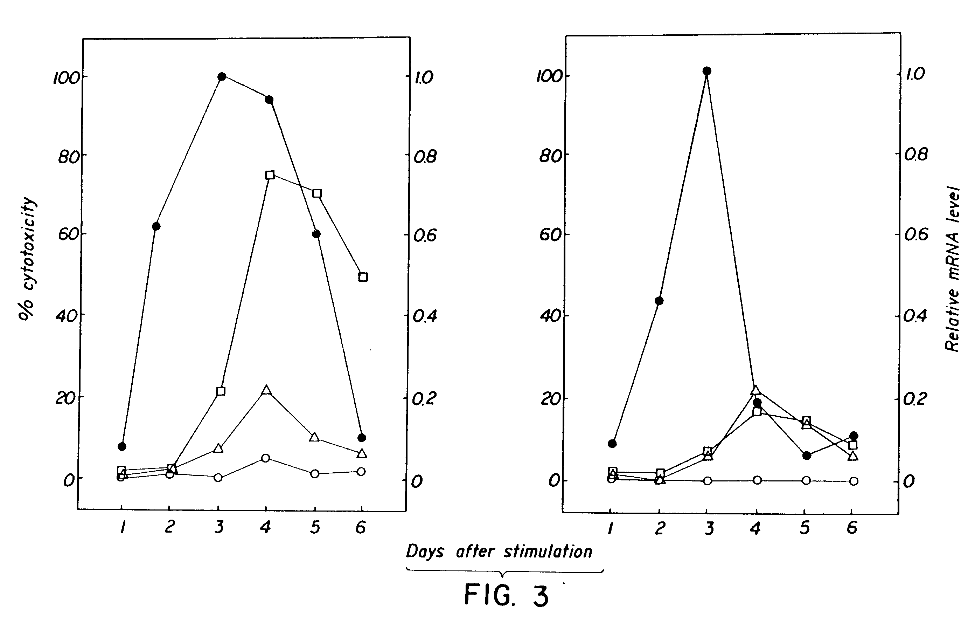

CBA/J (H-2^{k}) spleen cells were stimulated with either mitomycin C-treated EL4 cells (Fig. 3A) or Con A (Fig. 3B). On each of the 6 days after stimulation, the level of cytotoxicity was measured in a chromium-release assay against EL4 (H-2^{b}) (□), S194(H-2^{d}) (△), and RI(H-2^{k}) (○) cell lines. Cytodots were also prepared on each of these days, and the blots were hybridized with nick-translated B10 and C11. Again, data are presented only for B10, as C11 gave indistinguishable results. Relative B10 mRNA levels (●) were determined by scanning densitometry on an ELISA plate reader. In the allo-specific response (Fig. 3A), the peak of cytotoxicity was observed on day 4, while the peak of B10 or C11 mRNA expression appeared to be on days 3 and 4. The peak of killing activity in the Con A-stimulated cells (Fig. 3B) was also at day 4; however, the peak of mRNA expression was very sharply on day 3. In both experiments, the mRNA expression was reduced to background levels by day 6, while there were still significant levels of cytotoxicity on this day. When the cytodots were hybridized with ³²P-end-labeled oligo(dT), the peak of total mRNA was seen on day 2 (data not shown).

The experimental results illustrated in Fig. 3 indicates that the maximum expression of B10 and C11 mRNAs precedes the peak of cytotoxicity in an in vivo allogenic or mitogen-induced cytotoxic response by 24hrs; thus, they both fulfill the primary prerequisite for genes encoding proteins that are important in the lytic process.

### Example 2

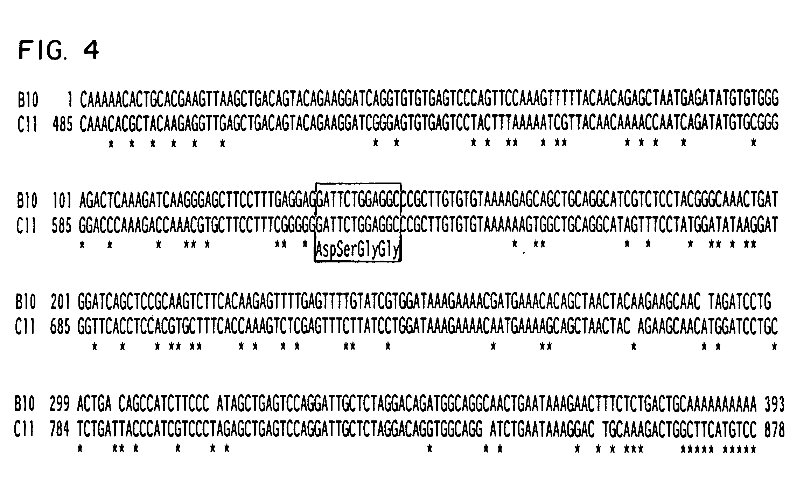

Clones B10 and C11 were sequenced according to the dideoxy method of Sanger et al., 143 J. Mol. Biol. 161 (1980). Sequence analysis of B10 and C11 (Fig. 4) reveals that they are related to each other and that the hypothetical proteins they encode contain a short region characteristic of serine proteases, Asp-Ser-Gly-Gly (a sequence homologous to that surrounding Ser¹⁹⁵ of chymotrypsin).

With B10 and C11 as probes, another CTL complementary DNA (cDNA) library was screened, in which inserts greater than 1000 base pairs were cloned in λgt10. Forty thousand recombinants were screened and 39 plaques corresponding to C11 were isolated.

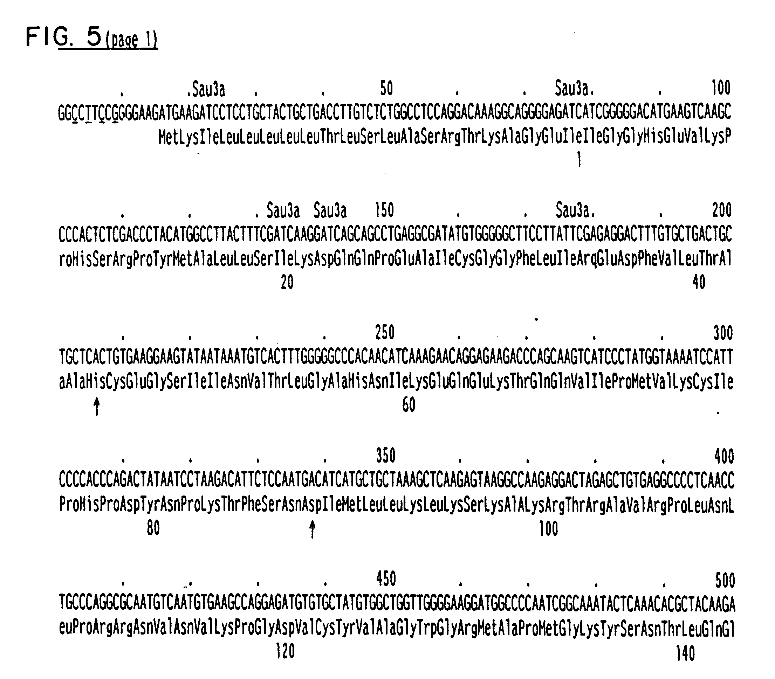

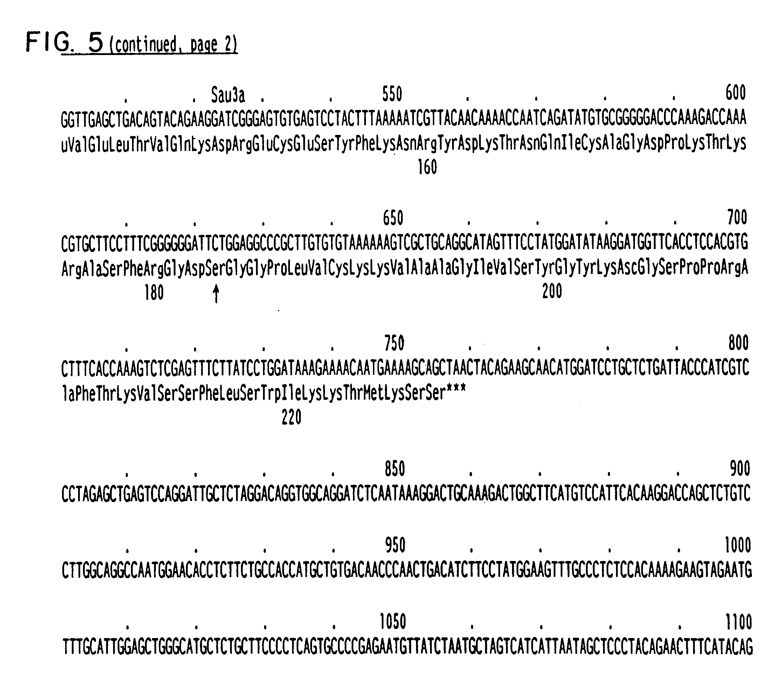

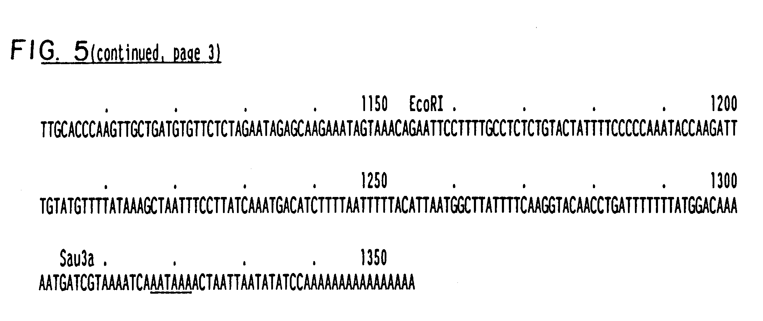

A cDNA insert of 1400 base pairs, which hybridized with C11, was selected for sequence analysis. The predicted protein sequence encoded, of molecular weight 25,319, is shown in Fig. 5. The putative start codon is preceded by a potential ribosome binding site CCUUCCG (Hagenbuchle et al., 13 Cell 551 (1978)), and a polyadenylation signal sequence AAUAAA (Proudfoot & Brownlee, 263 Nature 211 (1966)) occurs just upstream from the poly(A) tract. Of the first 12 amino acids predicted, ten are hydrophobic, and the amino acid in position 2 (Lys) is basic, suggesting that this sequence may act as a signal to direct secretion or intracellular organelle location. A search of the National Biomedical Research Foundation (NBRF) protein sequence data bank revealed that the protein encoded by C11 resembles a number of serine proteases (Table 1).

**Table 1**

| Protein | E.C. number | Species source | Residues Compared | | Percent homology |
|---|---|---|---|---|---|
| | | | CCPI | Bank Protein | |
| 7S nerve growth factor | 3.4.21 | Murine | 29-224 | 26-229 | 40 |
| Chymotrypsin A | 3.4.21.1 | Bovine | 1-200 | 16-216 | 35 |
| Chymotrypsin B | 3.4.21.1 | Bovine | 1-200 | 16-216 | 36 |
| Complement Clr | 3.4.21.41 | Human | 52-224 | 56-238 | 35 |
| Elastase | 3.4.21.11 | Porcine | 3-220 | 3-233 | 33 |
| Factor X | 3.4.21.6 | Bovine | 1-225 | 192-421 | 33 |
| RMCPII | 3.4.21 | Rat | 1-214 | 1-213 | 51 |
| Kallikrein | 3.4.21.8 | Rat | 26-225 | 51-262 | 36 |
| Plasminogen | 3.4.21.7 | Human | 3-224 | 563-787 | 37 |
| Plasminogen activator | 3.4.21.31 | Human | 72-224 | 389-560 | 35 |
| Trypsin | 3.2.21.4 | S. griseus | 29-220 | 22-214 | 33 |
| Trypsin | 3.2.21.4 | Rat | 29-226 | 31-228 | 39 |

When the sequences were optimally aligned according to the Dayhoff algorithm (Dayhoff, in 5 (Supp. 3) Atlas of Protein Sequencing and Structure 1 (National Biomedical Res. Found., Washington, D.C., 1979)), the homologies generally varied between 30 and 40 percent. The greatest homology was found with rat mast cell protease type II (RMCPII), which had amino acids identical to 109 of 215 amino acids encoded by C11, giving a match per length of 51 percent. The amino acid residues known to form the catalytic triad of the active site in serine proteases (His⁵⁷, Asp¹⁰², and Ser¹⁹⁵) were all found in the protein encoded by C11 (Fig. 5, △). The sequences around these residues, which are highly conserved among serine proteases, are also conserved in the C11 gene product. Indeed, largely because of conservation around this region, the protein encoded by C11 appears to be somewhat homologous (about 30 percent of 209 residues) even to the prokaryotic proteases trypsin and type B from Streptomyces griseus.

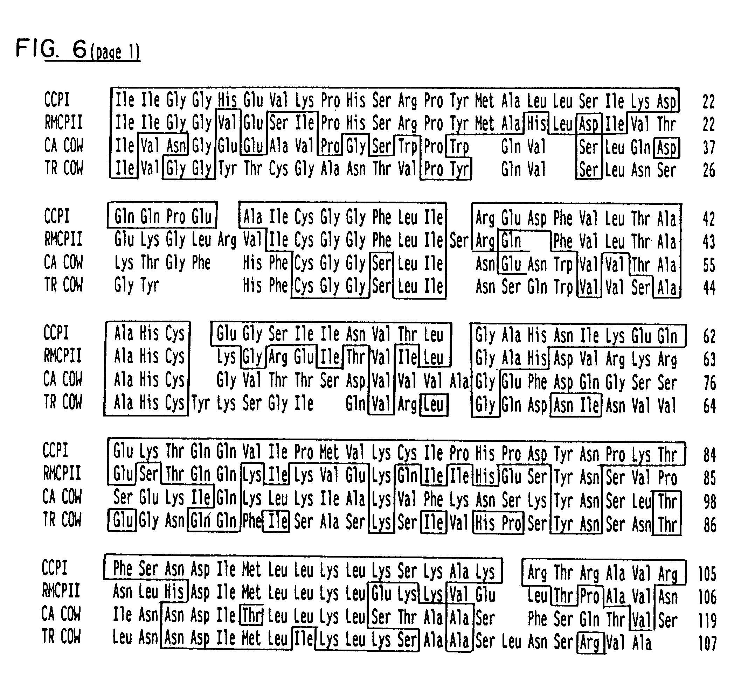

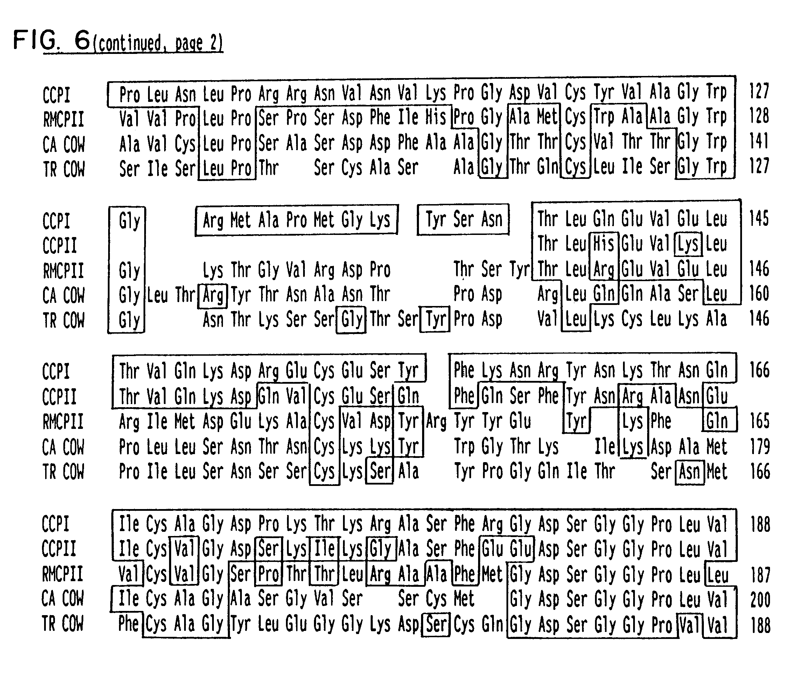

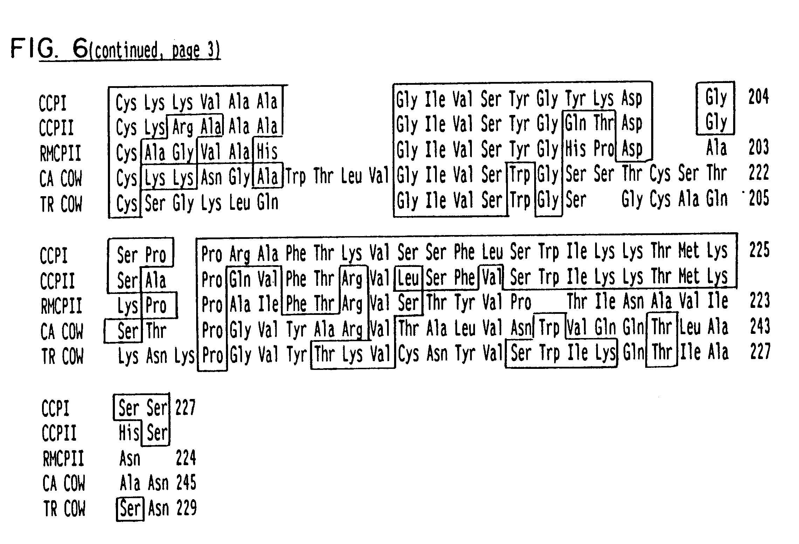

The cytotoxic T lymphocyte-specific proteins (CCP's) encoded by C11 and B10 Will be referred to as CCPI and CCPII, respectively. In Fig. 6 the optimal protein alignment with CCPI is presented for RMCPII, bovine chymotrypsin, bovine trypsin, and CCPII (not numbered, as the full sequence is not determined). RMCPII is an intracellular serine protease found in the granules of atypical mast cells. The high level of homology of CCPI with RMCPII is particularly intriguing as RMCPII has a number of structural features that make it exceptional in the serine protease superfamily. Protein CCPI contains cysteines in precisely the same positions as RMCPII which, by analogy with RMCPII, form three disulfide bonds. These occur in the same positions in chymotrypsin, trypsin, and elastase. Both CCPI and RMCPII lack a disulfide bond that is present in all other known serine proteases, including several from prokaryotes, and that links Cys¹⁹¹ with Cys²²⁰ in chymotrypsin. In both CCPI and RMCPII the first of these two half-cysteines is replaced by a phenylalanine, while the second half-cysteine has been deleted along with other residues. Linkage of Cys¹⁹¹ to Cys²²⁰ is thought to be important in stabilizing the conformation of the substrate binding site (Woodbury et al., 17 Biochem. 811 (1978)). Its absence in CCPI and RMCPII may lead to significant changes in that site and, hence, in substrate specificity.

Two other primary structure changes previously seen only in RMCPII and thought to alter substrate binding are also present in the predicted CCPI protein. In RMCPII and CCPI the amino acid six residues before the active-site serine is alanine. In chymotrypsin-like proteases it is serine and in trypsin-like proteases, aspartic acid. The residue in this position lies at the bottom of the S₁ binding site, so the change to a less polar residue would indicate a preference for a hydrophobic amino acid at the P₁ position in the substrate. Furthermore, the sequence Ser-Trp-Gly²¹⁶ in chymotrypsin, which forms hydrogen bonds with the P₁ and P₃ residues of the substrate, is replaced by Ser-Tyr-Gly in CCPI and RMCPII, again suggesting altered substrate specificity. Both of these changes are also seen with CCPII.

One of the few RMCPII-specific differences that is not present in CCPI is the substitution of isoleucine at position 99 in chymotrypsin for asparagine. In most mammalian serine proteases this residue is hydrophobic, and indeed in CCPI it appears to be phenylalanine. However, most of the RMCPII-specific changes are present in CCPI protein, suggesting that the substrate binding site of CCPI resembles that of RMCPII and is significantly different from those of other mammalian serine proteases.

### Example 3

The three-dimensional structure of the protease encoded by C11 was predicted by computer analysis. The active site has a serine residue at the back, meaning that it is a serine protease. Computer analysis indicated that this active site cleaves proteins at a cleavage site (between the C-linkage of an Asp residue and an N-linkage of an adjacent amino acid, Phe) different from the cleavage sites recognized by any other known serine proteases. This deduced cleavage site permits the synthesis of synthetic peptides which, by mimicking all or a portion of the natural cleavage site, can bind to the active site of the protease and competitively inhibit it.

### Example 4

The initial step in determining the structure of a protease expressed exclusively by human cytotoxic T lymphocytes and recognizing a unique protein cleavage site is to clone human cytotoxic T lymphocyte specific cDNAs.

PolyA⁺ RNA from a human cytotoxic T lymphocyte cell line, e.g., one of the lines on deposit at the Coriel Institute for Medical Research, Copewood and Davis Street, Camden, NJ, is used as a template for the synthesis, by standard procedures, of double stranded complementary DNA. EcoRI recognition sequences are then ligated onto the ends of the dscDNA by standard methods, and the resultant molecules are size selected on low melt agarose and then inserted into the EcoRI site of λgt11, all by conventional procedures. These recombinant molecules are then packaged into λ phage heads (Gigapack plus, Stragene) and used to infect E. coli Y1088. DNA from plaques harboring recombinant molecules are hybridized with radioactive probes generated from B10 and C11 by standard procedures to identify corresponding human genes. The screening is conducted in duplicate to minimize the possibility of false positives. A human counterpart of C11 was found using the above procedures.

The phage DNA from any positive plaques are isolated and immediately recloned, using conventional procedures, in the plasmid vector pUC13. Large amounts of these recombinant plasmid DNAs are then isolated for further analysis. The human cytotoxic T lymphocyte specific clones can be characterized by restriction enzyme digestions and, ultimately, sequence analysis. In addition, their relationships to one another can be investigated by standard cross-hybridization and heteroduplex mapping.

Tissue specific expression and transcript sizes of isolated genes can be established using the same methods as described for B10 and C11. The correlation between the level of cytotoxicity and the expression of the human genes also can be examined using the above-described methods.

When expression of a human cytotoxic T lymphocyte- specific gene correlates with toxicity, the gene is sequenced by standard methods (as was done with the B10 and C11 genes). From the gene sequence, the structure of the protease can be determined, and a computer analysis of the structure of protease performed, as with the C11 gene. Further computer analysis can show the location of the active site of the enzyme, and the appropriate sequence of a peptide that can act as a competitive inhibitor can be determined.

### Inhibitor Peptide Synthesis

The peptides of the invention can be prepared by standard solid phase synthesis, for example, a method in which a tert-butyloxycarbonylamino acid is attached to either chloromethyl resin containing 0.75 mM Cl⁻g⁻¹, or the p-methylbenzhydrylamine resin containing 0.35 mM NH₂ g⁻¹, followed by the sequential addition of desired amino acid residues to produce the desired peptide. Synthetic reactions are performed in 70 ml polypropylene syringes fitted with a polyethylene frit using apparatus and techniques described in Burton et al., 14 Biochemistry 3892 (1975), and Merrifield, 85 J. Amer. Chem. Soc. 2149 (1963). Completeness of coupling is determined by the standard ninhydrin test. The C-terminal amino acid is attached using procedures described in Stewart et al., Solid Phase Peptide Snythasis (W.H. Freeman ed. 1970), or Pietta et al., 1970 Chem. Comm. 650. Hplc purifications of the synthetic peptides are carried out using a Beckman ODS column (10 x 250 mm).

Amino acid analyses of the synthetic peptides are, if desired, performed using a Durrum D-500 analyzer. Cysteinyl residues in the peptides are quantitated as cysteic acid using a modification of the method of Moore (1968) in which 100 mM peptide is oxidized with 2.0 ml performic acid (1 ml 30% H₂O₂ + 9 ml 88% HCOOH) for 2 hrs. at 0°. Performic acid is removed in a Reacti-Therm at 40° using N₂, and 0.5 ml distilled water is then added to the residue and re-evaporated. The product is then hydrolyzed using 6 M HCl. Free sulfhydryl groups are determined using the method of Ellman et al. (1959).

### Use

The molecules are effective inhibitors of cytotoxic lymphocytes. The inhibition of the target cell destroying activity of such lymphocytes can be used to treat patients suffering from autoimmune diseases such as Hashimoto's thyroiditis, primary myxoedema, thyrotoxicosis, pernicious anaemia, autoimmune atrophic gastritis, Addison's disease, myasthenia gravis, juvenile diabetes, Goodpasture's syndrome, pemphigus vulgaris, pemphigoid, sympathetic ophthalmia, phacogenic uveitis, autoimmune haemolytic anaemia, idiopathic thrombocytopenic purpura, idiopathic leucopenia, primary biliary cirrhosis, active chronic hepatitis HB_{S}-ve, cryptogenic cirrhosis (some cases), ulcerative colitis, Sjögren's syndrome, systemic lupus erythematosus (SLE), discoid LE, dermatomyositis, scleroderma, rheumatoid arthritis, and possibly multiple sclerosis, and similar diseases in other mammals, for example, various types of livestock such as cows. Such inhibition can also be used to treat allograft (a tissue or organ graft from a donor who is a genetically dissimilar member of the same species as the receptor) rejection, and graft v. host disease.

The peptides can be administered to a mammal in a dosage of 25 to 500 mg/kg/day, preferably 50 to 100 mg/kg/day. When administered to mammals (e.g., orally, intravenously, parenterally, nasally, or by suppository), the peptides inhibit the ability of cytotoxic T lymphocytes to destroy cells, thus inhibiting the cell-mediated immune response to provide an effective treatment for the above listed disorders.

Nucleic acid probes (prepared by standard methods) capable of hybridizing to a gene encoding a protease expressed only by cytotoxic lymphocytes can be used in a variety of useful hybridization assays. For example, such probes can be used to monitor cytotoxic T lymphocytes in transplanted tissue, e.g., by the in situ hybridization methods of Cox et al., 101 Dev. Biol. 485 (1984). The presence of the lymphocytes in the transplanted tissue is an indication that the tissue is being rejected by the host organism and that appropriate immunotherapy should be undertaken.

The probes can also be used to assess the potential cytotoxicity of lymphokine activated killer cells. The generation and use of such cells to treat tumor patients is described by Rosenberg et al., 313 N.E.J. Med. 1485 (1985). Rosenberg describe how human peripheral-blood lymphocytes are treated with interleukin-2 (a lymphokine) to generate killer cells that will attack tumor cells when reintroduced into the host. The probes can be used in a hybridization assay with the nucleic acid of the treated lymphocytes by standard methods; the assay monitors the degree to which the activated killer cells have been generated by determining the level of expression of the protease-encoding gene in the cells.

### Other Embodiments

Other embodiments are within the following claims. For example, conventional computer analysis can be used to determine the structure of other molecules in addition to peptides that will bind to the active site of a particular protease and thus inhibit its activity. Examples of classes of molecules that are known to inhibit proteases include arsonic acids, sulphonyl fluorides, and organophosphorus compounds. Examples of inhibitors of serine proteases are listed by Powers & Harper in 12 Cell & Tissue Physiology 55 (Elsevier Science Publishers BV, 1986).

A gene encoding a cytotoxic lymphocyte-specific protease can be fused to a gene encoding a polypeptide ligand that specifically binds to the target cell (e.g., tumor cell), and the fused genes can be cloned into a vector that is then used to transform a microorganism, all by following the procedures described in Murphy, WO 83/03971. The resulting hybrid protein produced by the microorganisms will be capable of destroying the target cell when administered to a patient having a disease characterized by the presence of the unwanted cell.

Once an amino acid sequence for a protease has been determined, a small (e.g., 10-11 amino acid residue) region of the protein can be synthesized by standard techniques, and the synthesized peptide can be used as an antigen to raise antibodies, by conventional methods. The antibody can be used as a probe to detect the protease level in a sample, all by standard methods. The antibody-protease assay can be used as an alternative to any of the hybridization assays previously described.

Nucleic acid-containing samples include cell and tissue samples as well as nucleic acid isolated from such samples.

Conventional differential hybridization procedures, analogous to those used in the examples with mouse cell lines, can be used with human cytotoxic lymphocyte cell lines to isolate genes expressed only in the human cells.

## Claims

1. A peptide molecule capable of inhibiting the enzymatic activity of a protease produced by a cytotoxic lymphocyte in a mammal to which the molecule is administered, the peptide being incapable of inhibiting plasmin, the enzymatic activity of the protease being the ability to cleave a protein between the C-linkage of an aspartic acid residue of the protein and the N-linkage of an adjacent amino acid residue of the protein.

2. A peptide molecule as claimed in claim 1 wherein the protease is capable of cleaving a protein at a different site from a protease produced by a cell other than the cytotoxic lymphocyte cell.

3. A peptide molecule as claimed in claim 1 or 2 wherein the molecule is substantially incapable of inhibiting a protease produced by a non-cytotoxic lymphocyte cell.

4. A peptide molecule as claimed in any of claims 1 to 3 wherein the protease is a serine protease.

5. A peptide molecule as defined in any of claims 1 to 4 for use in medicine.

6. The use of a peptide molecule as defined in any of claims 1 to 4 in the preparation of an agent for the treatment of allograft rejection or an autoimmune disease or disorder.

7. A pharmaceutical composition comprising a peptide molecule capable of inhibiting the enzymatic activity of a protease produced by a cytotoxic lymphocyte in a mammal to which the molecule is administered, the peptide being incapable of inhibiting plasmin, and the enzymatic activity of the protease being the ability to cleave a protein between the C-linkage of an aspartic acid residue of the protein and the N-linkage of an adjacent amino acid residue of the protein;
and a pharmaceutically acceptable carrier.

8. A pharmaceutical composition as claimed in claim 7 wherein the protease is capable of cleaving a protein at a different site from a protease produced by a cell other than a cytotoxic lymphocyte cell.

## Patentansprüche

1. Peptidmolekül, das zur Hemmung der enzymatischen Aktivität einer Protease imstande ist, die durch einen cytotoxischen Lymphozyten in einem Säugetier erzeugt wird, dem das Molekül verabreicht wird, wobei das Peptid nicht imstande ist, Plasmin zu hemmen, und die enzymatische Aktivität der Protease die Fähigkeit ist, ein Protein zwischen der C-Bindung eines Asparaginsäurerestes des Proteins und der N-Bindung eines angrenzenden Aminosäurerestes des Proteins zu spalten.

2. Peptidmolekül nach Anspruch 1, wobei die Protease imstande ist, ein Protein an einer anderen Stelle zu spalten als eine Protease, die von einer anderen Zelle als der zytotoxischen Lymphozytenzelle erzeugt wird.

3. Peptidmolekül nach Anspruch 1 oder 2, wobei das Molekül im wesentlichen nicht imstande ist, eine Protease zu hemmen, die von einer nichtcytotoxischen Lymphozytenzelle erzeugt wird.

4. Peptidmolekül nach einem der Ansprüche 1 bis 3, wobei die Protease eine Serinprotease ist.

5. Peptidmolekül nach einem der Ansprüche 1 bis 4 zur Verwendung in der Medizin.

6. Verwendung eines Peptidmoleküls nach einem der Ansprüche 1 bis 4 in der Herstellung eines Mittels zur Behandlung einer Transplantatabstoßung oder einer autoimmunen Erkrankung oder Störung.

7. Pharmazeutische Zusammensetzung, welche ein Peptidmolekül, das zur Hemmung der enzymatischen Aktivität einer Protease imstande ist, die durch einen cytotoxischen Lymphozyten in einem Säugetier erzeugt wird, dem das Molekül verabreicht wird, wobei das Peptid nicht imstande ist, Plasmin zu hemmen, und die enzymatische Aktivität der Protease die Fähigkeit ist, ein Protein zwischen der C-Bindung eines Asparaginsäurerestes des Proteins und der N-Bindung eines angrenzenden Aminosäurerestes des Proteins zu spalten;
und einen pharmazeutisch annehmbaren Träger enthält.

8. Pharmazeutische Zusammensetzung nach Anspruch 7, wobei die Protease imstande ist, ein Protein an einer anderen Stelle zu spalten als eine Protease, die von einer anderen Zelle als der zytotoxischen Lymphozytenzelle erzeugt wird.

## Revendications

1. Molécule peptidique capable d'inhiber l'activité enzymatique d'une protéase produite par un lymphocyte cytotoxique chez un mammifère auquel la molécule est administrée, le peptide étant incapable d'inhiber la plasmine, l'activité de la protéase étant la capacité de cliver une protéine entre la liaison C d'un résidu acide aspartique de la protéine et la liaison N d'un résidu acide aminé adjacent de la protéine.

2. Molécule peptidique suivant la revendication 1, où la protéase est capable de cliver une protéine à un site différent de celui d'une protéase produite par une cellule autre que la cellule lymphocytaire cytotoxique.

3. Molécule peptidique suivant la revendication 1 ou 2, où la molécule est sensiblement incapable d'inhiber une protéase produite par une cellule lymphocytaire non cytotoxique.

4. Molécule peptidique suivant l'une quelconque des revendications 1 à 3, où la protéase est une protéase à sérine.

5. Molécule peptidique suivant l'une quelconque des revendications 1 à 4, à utiliser en médecine.

6. Utilisation d'une molécule peptidique telle que définie dans l'une quelconque des revendications 1 à 4, dans la préparation d'un agent pour le traitement des rejets d'allogreffes ou de maladies ou de désordres auto-immunitaires.

7. Composition pharmaceutique comprenant :
une molécule peptidique capable d'inhiber l'activité enzymatique d'une protéase produite par un lymphocyte cytotoxique chez un mammifère auquel la molécule est administrée, le peptide étant incapable d'inhiber la plasmine et l'activité de la protéase étant la capacité de cliver une protéine entre la liaison C d'un résidu acide résidu acide aminé adjacent de la protéine, et
un excipient pharmaceutiquement acceptable.

8. Composition pharmaceutique suivant la revendication 7, dans laquelle la protéase est capable de cliver une protéine à un site différent de celui d'une protéase produite par une cellule autre qu'une cellule lymphocytaire cytotoxique.
